(19) Europäisches Patentamt / European Patent Office / Office européen des brevets

(11) **EP 2 351 552 A1**

(12) **DEMANDE DE BREVET EUROPEEN**

(43) Date de publication:
**03.08.2011 Bulletin 2011/31**

(51) Int Cl.:
*A61K 8/06* *(2006.01)*          *A61K 8/31* *(2006.01)*
*A61Q 1/08* *(2006.01)*

(21) Numéro de dépôt: **10194890.9**

(22) Date de dépôt: **14.12.2010**

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**
Etats d'extension désignés:
**BA ME**

(30) Priorité: **18.12.2009  FR 0959221**

(71) Demandeur: **L'Oréal
75008 Paris (FR)**

(72) Inventeur: **Lebre-Lemonnier, Caroline
75005, Paris (FR)**

(74) Mandataire: **Leonard, Armelle
L'Oréal
D.I.P.I.
25-29, Quai Aulagnier
92665 Asnières-sur-Seine Cedex (FR)**

(54)     **Composition colorée pour camoufler les imperfections cutanées**

(57)     La présente invention concerne une composition cosmétique colorée de maquillage des matières kératiniques, en particulier la peau, sous la forme d'une émulsion eau-dans huile comprenant, dans un milieu physiologiquement acceptable:

(i) au moins 18% en poids de matières colorantes pulvérulentes, par rapport au poids total de la composition,
(ii) au moins un ou plusieurs alcanes linéaires volatils notamment en C7-C14.

**Description**

La présente invention a pour objet une composition cosmétique colorée de maquillage des matières kératiniques, en particulier de la peau, sous la forme d'une émulsion eau-dans-huile apte à camoufler les imperfections colorielles desdites matières kératiniques. Il s'agira en particulier de fonds de teint ou correcteurs de teint.

Par composition colorée, on entend une composition non blanche.

L'obtention d'un résultat maquillage permettant de camoufler des imperfections cutanées, telles que des taches ou des zones hypo ou hyper pigmentées, passe généralement par l'incorporation d'un fort taux de pigments, qui se traduit bien souvent par des propriétés d'application indésirables : la texture de la composition devient pâteuse et donc difficile à étaler. Le maquillage obtenu n'est pas homogène, des zones de couleurs différentes apparaissent sur la peau, rendant le maquillage inesthétique. De plus, la texture pâteuse est inconfortable pour l'utilisatrice pendant et après l'application de la composition sur la peau.

Bien que les huiles siliconées aient permis il y a une vingtaine d'années de faire progresser la sensorialité des produits de type fonds de teint, la réalisation de tels produits, couvrants mais agréables à appliquer, se révèle souvent difficile.

La réalisation d'émulsions pigmentées à fort pouvoir couvrant représente en particulier un défi technique délicat à relever pour le formulateur. En effet, haute couvrance et propriétés cosmétiques agréables sont bien souvent 2 paramètres antagonistes difficiles à concilier.

D'autant que le taux élevé de pigments accentue les problèmes de stabilité de la composition lors de son stockage au cours du temps, et ceci est d'autant plus vrai que la composition est sous la forme d'une émulsion. En effet, les pigments ont tendance à sédimenter et un surnageant important d'huiles peut apparaître. La composition présente alors un déphasage et n'est plus homogène : elle ne permet pas d'obtenir un maquillage homogène et son aspect devient particulièrement inesthétique.

Il est souvent plus aisé d'obtenir un film de maquillage opacifiant via une galénique anhydre. Celle-ci présente cependant l'inconvénient de ne pas permettre l'incorporation aisée des actifs utilisés en cosmétiques (actifs anti-rides, hydratants, destinés aux peaux grasses...), le plus souvent solubilisés dans la phase aqueuse de la préparation. Les produits de teint anhydres ne sont par ailleurs pas appréciés par tout type de consommatrices étant donné leur caractère parfois gras ou huileux.

Il existe donc un besoin de disposer de compositions de maquillage permettant de camoufler efficacement les défauts de couleurs de la peau (en particulier les taches et les zones hypo ou hyper pigmentaires) et présentant une texture permettant une application facile sur la peau.

La Demanderesse a découvert que l'utilisation d'un ou plusieurs alcanes linéaires volatils en C7-C14, dans la phase huileuse d'une émulsion eau-dans-huile, permettait d'incorporer une teneur élevée en pigments dans lesdites émulsions (voire des teneurs supérieures à 25% en poids), et donnait au produit obtenu des propriétés cosmétiques améliorées à l'application (ce que ne permet pas toujours l'utilisation d'huiles siliconées).

De façon plus précise, la présente invention vise une composition de maquillage des matières kératiniques, en particulier la peau, sous la forme d'une émulsion eau-dans-huile, permettant de couvrir efficacement et de manière homogène les imperfections cutanées, notamment les défauts de couleur de la peau, tout en conservant une texture fluide, agréable à utiliser et conduisant à un maquillage présentant une bonne couvrance.

L'invention a pour objet, selon un premier aspect, une composition cosmétique colorée de maquillage des matières kératiniques, notamment destinée à camoufler les imperfections cutanées, en particulier les défauts de couleur de la peau, sous la forme d'une émulsion eau-dans huile comprenant, dans un milieu physiologiquement acceptable:

    (i) au moins 18% en poids de matières colorantes pulvérulentes, par rapport au poids total de la composition,

    (ii) au moins un ou plusieurs alcane(s) linéaire(s) volatil(s) notamment en C7-C14.

Lesdites matières colorantes pulvérulentes utilisées selon l'invention ont une taille supérieure ou égale à 100nm.

En particulier, lesdites matières colorantes pulvérulentes sont choisies parmi des particules ayant une taille allant de 100 nm à 10 $\mu$m, de préférence de 200 nm à 5 $\mu$m, et plus préférentiellement de 250 nm et 1 $\mu$m.

De préférence, lesdites matières colorantes pulvérulentes comprennent au moins des particules colorées (non blanches), en particulier des oxydes de fer.

Selon un mode préféré, les matières pulvérulentes comprennent des oxydes de fer et des oxydes de titane.

En particulier, le ou les alcanes linéaires volatiles notamment en C7-C14 représentent au moins 50% en poids, notamment au moins 65% en poids, en particulier au moins 80% en poids, voire 100% en poids, par rapport au poids total des huiles de la composition.

Selon un mode particulier, la composition de l'invention comprend au moins 20% en poids, notamment au moins 25% en poids, voire au moins 30% en poids de matières colorantes pulvérulentes par rapport au poids total de ladite composition.

La composition selon l'invention est notamment un fond de teint ou un correcteur de teint. Elle peut être appliquée de façon uniforme sur la peau du visage, ou par petites touches pour camoufler des imperfections cutanées. Il s'agira alors d'un correcteur de teint.

La composition de l'invention présente généralement une couvrance supérieure à 50, de préférence supérieure à 60 et mieux supérieure à 70. En particulier, elle présente une couvrance allant de 70 à 100, en particulier de 80 à 100 et mieux de 90 à 100.

Mesure de couvrance

Les supports utilisés pour la mesure de couvrance selon l'invention sont des plaques de Bioskin de couleur noire et blanche (fournisseur Maprecos). Les compositions selon l'invention sont appliquées au doigtier à raison de 32mg sur une surface de 5cm de diamètre, puis séchés pendant 90 minutes. En particulier, l'épaisseur appliquée est de 15$\mu$m.

Les spectres de réflectance sont acquis à l'aide d'un spectrocolorimètre MINOLTA 3700-d (géométrie de mesure diffuse et observation D65/10°, mode composante spéculaire exclue) sur les fonds noir et blanc (3 mesures sur chaque application).

Les spectres sont exprimés en coordonnées colorimétriques dans l'espace CIEYxy1931 au sens de la Commission Internationale de l'Eclairage.

Le contraste ratio CR, ou couvrance, est calculé en faisant la moyenne arithmétique de Y sur fond noir, divisée par la valeur moyenne de Y sur fond blanc, multiplié par 100.

La présente invention vise également un procédé cosmétique de maquillage des matières kératiniques, en particulier la peau, comprenant l'application d'une composition selon l'invention.

En particulier, ledit procédé est destiné à camoufler les imperfections cutanées de la peau, notamment les défauts de couleur de la peau.

La composition selon l'invention est sous la forme d'une émulsion eau-dans-huile, comprenant une phase aqueuse dispersée dans une phase grasse liquide, et comprend au moins un milieu physiologiquement acceptable.

Par « milieu physiologiquement acceptable », on entend désigner un milieu convenant particulièrement à l'application d'une composition de l'invention sur les matières kératiniques, notamment la peau et les lèvres.

Le milieu physiologiquement acceptable est généralement adapté à la nature du support sur lequel doit être appliquée la composition, ainsi qu'à l'aspect sous lequel la composition doit être conditionnée.

La composition selon l'invention comprend au moins 18% en poids de matières colorantes pulvérulentes, par rapport au poids total de ladite composition.

**MATIERES COLORANTES PULVERULENTES**

Les matières colorantes pulvérulentes peuvent être présentes à raison de 18% à 70 % en poids, notamment, de 20 à 50 % en poids, et en particulier, de 25 à 35 % en poids, par rapport au poids total de la composition cosmétique.

Selon un mode préféré, les matières colorantes pulvérulentes comprennent au moins des particules colorées (non blanches).

Les matières colorantes pulvérulentes sont notamment choisies parmi les pigments, les nacres, les matériaux à effet optique spécifique, et leurs mélanges. En particulier, il s'agira de pigments.

Pigments

Par pigments, il faut comprendre des particules blanches ou colorées, minérales ou organiques, insolubles dans une solution aqueuse, destinées à colorer et/ou opacifier le film résultant.

En particulier, la composition selon l'invention comprend au moins des particules colorées, notamment des oxydes de fer.

Comme pigments minéraux utilisables dans l'invention, on peut citer les oxydes de titane, de zirconium ou de cérium, ainsi que les oxydes de zinc, de fer ou de chrome, le bleu ferrique, le violet de manganèse, le bleu outremer et l'hydrate de chrome.

On utilise de préférence des pigments d'oxydes de fer, de dioxyde de titane, et leurs mélanges.

Il peut également s'agir de pigment ayant une structure qui peut être par exemple de type séricite/oxyde de fer brun/dioxyde de titane/silice. Un tel pigment est commercialisé par exemple sous la référence COVERLEAF NS ou JS par la société CHEMICALS AND CATALYSTS et présente un rapport de contraste voisin de 30.

La matière colorante peut encore comporter un pigment ayant une structure qui peut être, par exemple, de type microsphères de silice contenant de l'oxyde de fer. Un exemple de pigment présentant cette structure est celui commercialisé par la société MIYOSHI sous la référence PC BALL PC-LL-100 P, ce pigment étant constitué de microsphères de silice contenant de l'oxyde de fer jaune.

Parmi les pigments organiques utilisables dans l'invention, on peut citer le noir de carbone, les pigments de type D & C, les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Avantageusement, les pigments peuvent être présents sous une forme enrobée hydrophobe dans l'émulsion selon l'invention. Il s'agit plus particulièrement de pigments traités en surface avec un agent hydrophobe pour les rendre compatibles avec la phase grasse de l'émulsion, notamment pour qu'ils aient une bonne mouillabilité avec les huiles de la phase grasse. Ainsi, ces pigments traités sont bien dispersés dans la phase grasse.

L'agent de traitement hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl silanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.

Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zinc, de sodium, de potassium. L'acide aminé peut être par exemple la lysine, l'acide glutamique, l'alanine.

Le terme alkyle mentionné dans les composés cités précédemment désigne notamment un groupe alkyle ayant de 1 à 30 atomes de carbone, de préférence ayant de 5 à 16 atomes de carbone.

Des pigments traités hydrophobes sont notamment décrits dans la demande EP-A-1086683.

Nacres

Par « nacres », il faut comprendre des particules colorées de toute forme, irisées ou non, notamment, produites par certains mollusques dans leur coquille ou bien synthétisées et qui présentent un effet de couleur par interférence optique.

Les nacres peuvent être choisies parmi les pigments nacrés, tels que le mica titane recouvert avec un oxyde de fer, le mica titane recouvert avec de l'oxychlorure de bismuth, le mica titane recouvert avec de l'oxyde de chrome, le mica titane recouvert avec un colorant organique, ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Matériaux à effet optique

La composition cosmétique selon l'invention peut également contenir au moins un matériau à effet optique spécifique.

Cet effet est différent d'un simple effet de teinte conventionnel, c'est-à-dire unifié et stabilisé tel que produit par les matières colorantes classiques, comme, par exemple, les pigments monochromatiques. Au sens de l'invention, « stabilisé » signifie dénué d'effet de variabilité de la couleur avec l'angle d'observation ou encore en réponse à un changement de température.

Par exemple, ce matériau peut être choisi parmi les particules à reflet métallique, les agents de coloration goniochromatiques, les pigments diffractants, les agents thermochromes, les agents azurants optiques, ainsi que les fibres, notamment, interférentielles. Bien entendu, ces différents matériaux peuvent être associés de manière à procurer la manifestation simultanée de deux effets, voire d'un nouvel effet conforme à l'invention.

Les particules à reflet métallique utilisables dans l'invention sont en particulier choisies parmi :

- les particules d'au moins un métal et/ou d'au moins un dérivé métallique,
- les particules comportant un substrat organique ou minéral, monomatière ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple, les bronzes et les laitons) sont des métaux préférés.

Par « dérivés métalliques », on désigne des composés dérivés de métaux, notamment, des oxydes, des fluorures, des chlorures et des sulfures

L'agent de coloration goniochromatique peut être choisi, par exemple, parmi les structures multicouches interférentielles et les agents de coloration à cristaux liquides.

La composition selon l'invention comprend au moins un ou plusieurs alcanes linéaires volatils notamment en C7-C14.

## ALCANES LINEAIRES VOLATILS

La composition de l'invention peut comprendre de 5 % à 50 % en poids d'alcane(s) linéaire(s) volatil(s), en particulier de 8 à 40 % en poids d'alcane(s) linéaire(s) volatil(s), et plus particulièrement de 10 à 30 % en poids d'alcane(s) linéaire(s) volatil(s) notamment en C7-C14 par rapport au poids total de la composition.

Selon un mode particulier de réalisation, la composition de l'invention peut comprendre au moins 50 % en poids d'alcane(s) linéaire(s) volatil(s) par rapport à la teneur totale en huile(s) volatile(s) de la composition. En particulier, une composition de l'invention peut comprendre au moins 60 %, plus particulièrement au moins 70 %, et plus particulièrement au moins 80 %, au moins 90 % ou 100 % d'alcane(s) linéaire(s) volatil(s) par rapport à la teneur totale en huile (s) volatile(s) de la composition.

Une composition de l'invention comprenant 100 % d'alcane(s) linéaire(s) volatil(s) par rapport à la teneur totale en huile (s) volatile(s) comprend une phase huileuse volatile composée exclusivement d'alcane(s) linéaire(s) volatil(s).

Par « huile », on entend un composé non aqueux, non miscible à l'eau, liquide à température ambiante (25°C) et pression atmosphérique (760 mm de Hg).

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor ; elle peut contenir des groupes ester, éther, amine, amide.

Par « un ou plusieurs alcane(s) linéaire(s) volatil(s) », on entend indifféremment « une ou plusieurs huile alcane (s) linéaire(s) volatile(s) ».

Un alcane linéaire volatil convenant à l'invention est liquide à température ambiante (environ 25 °C) et à la pression atmosphérique (760 mm Hg).

Par « alcane linéaire volatil » convenant à l'invention, on entend un alcane linéaire cosmétique, susceptible de s'évaporer au contact de la peau en moins d'une heure, à température ambiante (25°C) et pression atmosphérique (760 mm Hg, c'est-à-dire 101 325 Pa), liquide à température ambiante, ayant notamment une vitesse d'évaporation allant de 0,01 à 15 mg/cm$^2$/min, à température ambiante (25°C) et pression atmosphérique (760 mm Hg).

De façon préférée, les « alcanes linéaires volatils » convenant à l'invention présentent une vitesse d'évaporation allant de 0,01 à 3,5 mg/cm$^2$/min, à température ambiante (25°C) et pression atmosphérique (760 mm Hg).

De façon préférée, les « alcanes linéaires volatils » convenant à l'invention présentent une vitesse d'évaporation allant de 0,01 à 1,5 mg/cm$^2$/min, à température ambiante (25°C) et pression atmosphérique (760 mm Hg).

De façon plus préférée, les « alcanes linéaires volatils » convenant à l'invention présentent une vitesse d'évaporation allant de 0,01 à 0,8 mg/cm$^2$/min, à température ambiante (25°C) et pression atmosphérique (760 mm Hg).

De façon encore préférée, les « alcanes linéaires volatils » convenant à l'invention présentent une vitesse d'évaporation allant de 0,01 à 0,3 mg/cm$^2$/min, à température ambiante (25°C) et pression atmosphérique (760 mm Hg).

De façon encore préférée, les « alcanes linéaires volatils » convenant à l'invention présentent une vitesse d'évaporation allant de 0,01 à 0,12 mg/cm$^2$/min, à température ambiante (25°C) et pression atmosphérique (760 mm Hg).

La vitesse d'évaporation d'un alcane volatil conforme à l'invention (et plus généralement d'un solvant volatil) peut être notamment évaluée au moyen du protocole décrit dans WO 06/013413, et plus particulièrement au moyen du protocole décrit ci-après.

On introduit dans un cristallisoir (diamètre : 7 cm) placé sur une balance se trouvant dans une enceinte d'environ 0,3 m$^3$ régulée en température (25 °C) et en hygrométrie (humidité relative 50 %) 15 g de solvant hydrocarboné volatil.

On laisse le liquide s'évaporer librement, sans l'agiter, en assurant une ventilation par un ventilateur (PAPST-MOTOREN, référence 8550 N, tournant à 2700 tours/minute) disposé en position verticale au-dessus du cristallisoir contenant le solvant hydrocarboné volatil, les pales étant dirigées vers le cristallisoir, à une distance de 20 cm par rapport au fond du cristallisoir.

On mesure à intervalles de temps réguliers la masse de solvant hydrocarboné volatil restante dans le cristallisoir.

On obtient alors le profil d'évaporation du solvant en traçant la courbe de la quantité de produit évaporé (en mg/cm$^2$) en fonction du temps (en min).

Puis on calcule la vitesse d'évaporation qui correspond à la tangente à l'origine de la courbe obtenue. Les vitesses d'évaporation sont exprimées en mg de solvant volatil évaporé par unité de surface (cm$^2$) et par unité de temps (minute).

Selon un mode de réalisation préféré, les « alcanes linéaires volatils » convenant à l'invention ont une pression de vapeur (appelée également pression de vapeur saturante) non nulle, à température ambiante, en particulier une pression de vapeur allant de 0,3 Pa à 6000 Pa.

De façon préférée, les « alcanes linéaires volatils » convenant à l'invention ont une pression de vapeur allant de 0,3 à 2000 Pa, à température ambiante (25°C).

De façon préférée, les « alcanes linéaires volatils » convenant à l'invention ont une pression de vapeur allant de 0,3 à 1000 Pa, à température ambiante (25°C)

De façon plus préférée, les « alcanes linéaires volatils » convenant à l'invention ont une pression de vapeur allant de 0,4 à 600 Pa, à température ambiante (25°C).

De façon préférée, les « alcanes linéaires volatils » convenant à l'invention ont une pression de vapeur allant de 1 à 200 Pa, à température ambiante (25°C).

De façon encore préférée, les « alcanes linéaires volatils » convenant à l'invention ont une pression de vapeur allant de 3 à 60 Pa, à température ambiante (25°C).

Selon un mode de réalisation, un alcane linéaire volatil convenant à l'invention peut présenter un point éclair compris dans l'intervalle variant de 30 à 120 °C, et plus particulièrement de 40 à 100 °C. Le point éclair est en particulier mesuré selon la Norme iso 3679.

Selon un mode de réalisation, un alcane convenant à l'invention peut être un alcane linéaire volatil comprenant de 7 à 14 atomes de carbone.

De façon préférée, les « alcanes linéaires volatils » convenant à l'invention comprennent de 8 à 14 atomes de carbone.

De façon préférée, les « alcanes linéaires volatils » convenant à l'invention comprennent de 9 à 14 atomes de carbone.

De façon préférée, les « alcanes linéaires volatils » convenant à l'invention comprennent de 10 à 14 atomes de carbone.

De façon préférée, les « alcanes linéaires volatils » convenant à l'invention comprennent de 11 à 14 atomes de carbone.

Selon un mode de réalisation avantageux, les « alcanes linéaires volatils » convenant à l'invention présentent une vitesse d'évaporation, telle que définie plus haut, allant de 0,01 à 3,5 mg/cm$^2$/min, à température ambiante (25°C) et pression atmosphérique (760 mm Hg), et comprennent de 8 à 14 atomes de carbone.

Un alcane linéaire volatil convenant à l'invention peut être avantageusement d'origine végétale.

De préférence, l'alcane linéaire volatil ou le mélange d'alcanes linéaires volatils présent dans la composition selon l'invention comprend au moins un isotope $^{14}$C du carbone (carbone 14), en particulier l'isotope $^{14}$C peut être présent en un ratio $^{14}$C / $^{12}$C supérieur ou égal à $1.10^{-16}$, de préférence supérieur ou égal à $1.10^{-15}$, de préférence encore supérieur ou égal $7,5.10^{-14}$, et mieux supérieur ou égal $1,5.10^{-13}$. De préférence, le ratio $^{14}$C / $^{12}$C va de $6.10^{-13}$ à $1,2.10^{-12}$.

La quantité de d'isotopes $^{14}$C dans l'alcane linéaire volatil ou le mélange d'alcanes linéaires volatils peut être déterminée par des méthodes connues de l'homme du métier telles que la méthode de comptage de Libby, la spectrométrie à scintillation liquide ou encore la spectrométrie de masse à accélération (Accelerator Mass Spectrometry).

Un tel alcane peut être obtenu, directement ou en plusieurs étapes, à partir d'une matière première végétale comme une huile, un beurre, une cire, etc.

A titre d'exemple d'alcanes convenant à l'invention, on peut mentionner les alcanes décrits dans les demandes de brevets de la société Cognis WO 2007/068371, ou WO2008/155059 (mélanges d'alcanes distincts et différant d'au moins un carbone). Ces alcanes sont obtenus à partir d'alcools gras, eux-mêmes obtenus à partir d'huile de coprah ou de palme.

A titre d'exemple d'alcanes linéaires convenant à l'invention, on peut citer le n- heptane (C7), le n-octane (C8), le n-nonane (C9), le n-décane (C10), le n-undécane (C11), le n-dodécane (C12), le n-tridécane (C13), le n-tétradecane (C14), et leurs mélanges. Selon un mode de réalisation particulier, l'alcane linéaire volatil est choisi parmi le n-nonane, le n-undécane, le n-dodécane, le n-tridécane, le n-tétradécane, et leurs mélanges.

Selon un mode préféré, on peut citer les mélanges de n-undécane (C11) et de n-tridécane (C13) obtenus aux exemples 1 et 2 de la demande WO2008/155059 de la Société Cognis.

On peut également citer le n-dodécane (C12) et le n-tétradécane (C14) vendus par Sasol respectivement sous les références PARAFOL 12-97 et PARAFOL 14-97, ainsi que leurs mélanges.

On pourra utiliser l'alcane linéaire volatil seul.

On pourra alternativement ou préférentiellement utiliser un mélange d'au moins deux alcanes linéaires volatils distincts, différant entre eux d'un nombre de carbone n d'au moins 1, en particulier différant entre eux d'un nombre de carbone de 1 ou de 2.

Selon un premier mode de réalisation, on utilise un mélange d'au moins deux alcanes linéaires volatils distincts comportant de 10 à 14 atomes de carbone et différant entre eux d'un nombre de carbone d'au moins 1. A titre d'exemples, on peut citer notamment les mélanges d'alcanes linéaires volatils C10/C11, C11/C12, ou C12/C13.

Selon un autre mode de réalisation, on utilise un mélange d'au moins deux alcanes linéaires volatils distincts comportant de 10 à 14 atomes de carbone et différant entre eux d'un nombre de carbone d'au moins 2. A titre d'exemples, on peut citer notamment les mélanges d'alcanes linéaires volatils C10/C12, ou C12/C14, pour un nombre de carbone n pair et le mélange C11/C13 pour un nombre de carbone n impair.

Selon un mode préféré, on utilise un mélange d'au moins deux alcanes linéaires volatils comportant de 10 à 14 atomes de carbone distincts et différant entre eux d'un nombre de carbone d'au moins 2, et en particulier un mélange d'alcanes linéaires volatils C11/C13 ou un mélange d'alcanes linéaires volatils C12/C14.

D'autres mélanges associant plus de 2 alcanes linéaires volatils selon l'invention, tels que par exemple un mélange d'au moins 3 alcanes linéaires volatils comportant de 7 à 14 atomes de carbone distincts et différant entre eux d'un nombre de carbone d'au moins 1, font également partie de l'invention, mais les mélanges de 2 alcanes linéaires volatils selon l'invention sont préférés (mélanges binaires), lesdits 2 alcanes linéaires volatils représentant de préférence plus de 95% et mieux plus de 99% en poids de la teneur totale en alcanes linéaires volatils dans le mélange.

Selon un mode particulier de l'invention, dans un mélange d'alcanes linéaires volatils, l'alcane linéaire volatil ayant le nombre de carbone le plus petit est majoritaire dans le mélange.

Selon un autre mode de l'invention, on utilise un mélange d'alcanes linéaires volatils dans lequel l'alcane linéaire volatil ayant le nombre de carbone le plus grand est majoritaire dans le mélange.

A titre d'exemples de mélanges convenant à l'invention, on peut citer notamment les mélanges suivants :

- de 50 à 90% en poids, de préférence de 55 à 80% en poids, préférentiellement encore de 60 à 75% en poids d'alcane linéaire volatil en Cn avec n allant de 7 à 14
- de 10 à 50% en poids, de préférence de 20 à 45% en poids, de préférence de 24 à 40% en poids, d'alcane linéaire volatil en Cn+x avec x supérieur ou égal à 1, de préférence x=1 ou x=2, avec n+x compris entre 8 et 14, par rapport au poids total des alcanes dans ledit mélange.

En particulier, ledit mélange d'alcanes selon l'invention contient :

- moins de 2% en poids, de préférence moins de 1% en poids d'hydrocarbures ramifiés,
- et/ou moins de 2% en poids, de préférence moins de 1% en poids d'hydrocarbures aromatiques,
- et/ou moins de 2% en poids, de préférence moins de 1% en poids et préférentiellement moins de 0.1% en poids d'hydrocarbures insaturés dans le mélange.

Plus particulièrement, un alcane linéaire volatil convenant à l'invention peut être mis en oeuvre sous la forme d'un mélange n-undécane/n-tridécane.

En particulier, on utilisera un mélange d'alcanes linéaires volatils comprenant :

- de 55 à 80% en poids, de préférence de 60 à 75% en poids d'alcane linéaire volatil en C11 (n-undécane)
- de 20 à 45% en poids, de préférence de 24 à 40% en poids d'alcane linéaire volatil en C13 (n-tridécane) par rapport au poids total des alcanes dans ledit mélange.

Selon un mode de réalisation particulier, le mélange d'alcanes est un mélange n-undécane/n-tridécane. En particulier un tel mélange peut être obtenu selon l'exemple 1 ou l'exemple 2 du WO 2008/155059.

Selon un autre mode de réalisation particulier, on utilise le n-dodécane vendu sous la référence PARAFOL 12-97 par SASOL.

Selon un autre mode de réalisation particulier, on utilise le n-tétradécane vendu sous la référence PARAFOL 14-97 par SASOL.

Selon encore un autre mode de réalisation, on utilise un mélange de n-dodécane et de n-tétradécane.

Selon un mode particulier de l'invention, la composition peut comprendre moins de 10% en poids, voire moins de 5% en poids, voire moins de 2% en poids, voire être dépourvue d'huile siliconée cyclique.

**GALENIQUE**

La composition selon l'invention, sous la forme d'une émulsion eau-dans-huile, est caractérisée notamment par une phase aqueuse dispersée dans une phase grasse liquide, généralement un tensioactif et la présence éventuellement d'au moins un agent épaississant destiné à augmenter la viscosité de ladite composition.

**_PHASE AQUEUSE_**

Une composition selon l'invention comprend au moins une phase aqueuse, de préférence en une teneur allant de 10 à 80 %, et plus particulièrement de 25 à 70 % en poids par rapport au poids total de la composition.

La phase aqueuse comprend de l'eau et/ou au moins un solvant hydrosoluble.

L'eau peut être une eau florale telle que l'eau de bleuet et/ou une eau minérale telle que l'eau de VITTEL, l'eau de LUCAS ou l'eau de LA ROCHE POSAY et/ou une eau thermale.

Par « solvant hydrosoluble », on désigne dans la présente invention un composé liquide à température ambiante et miscible à l'eau (miscibilité dans l'eau supérieure à 50 % en poids à 25 °C et pression atmosphérique).

Le ou les solvant(s) hydrosolubles convenant à l'invention peu(vent)t être choisi(s) parmi les monoalcools en $C_{1-8}$, et notamment en $C_{1-5}$, notamment l'éthanol, l'isopropanol, le tert-butanol, le n-butanol, les polyols tels que décrits précédemment, et leurs mélanges. Conviennent aussi tout particulièrement à l'invention, l'éthanol et l'isopropanol et de préférence l'éthanol.

Une composition de l'invention peut en outre comprendre au moins un sel, par exemple, le chlorure de sodium, le chlorure de magnésium et le sulfate de magnésium.

Une composition de l'invention peut comprendre de 0,05 % à 1,5 % en particulier de 0,1 % à 1,0 % et plus particulièrement de 0,15 % à 0,8 % en poids de sels, par rapport au poids total de la composition.

La phase aqueuse peut également comprendre tout composé hydrosoluble ou hydrodispersible compatible avec une phase aqueuse tels que des gélifiants, des polymères filmogènes, des épaississants, des tensio-actifs et leurs mélanges.

## *PHASE GRASSE LIQUIDE*

Une composition cosmétique conforme à la présente invention comprend au moins une phase grasse liquide contenant au moins un alcane linéaire volatil notamment en C7-C14 tel que défini précédemment, et éventuellement au moins une huile additionnelle et/ou corps gras additionnels.

Une composition de l'invention peut comprendre une phase grasse liquide en une teneur variant de 10 à 90 %, en particulier de 15 à 60 %, et plus particulièrement, de 20 à 50 % en poids par rapport au poids total de la composition.

### Huiles additionnelles

Une composition selon l'invention peut comprendre au moins une huile additionnelle, choisie parmi les huiles volatiles et non volatiles de type hydrocarbonées, siliconées, ou fluorées. Les huiles peuvent être d'origine animale, végétale, minérale ou synthétique.

On entend par huile, tout corps gras sous forme liquide à température ambiante (20-25 °C) et à pression atmosphérique.

Par "huile volatile", on entend une huile (ou milieu non aqueux) susceptible de s'évaporer au contact de la peau en moins d'une heure, à température ambiante et pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa ($10^{-3}$ à 300 mm de Hg), et de préférence allant de 1,3 Pa à 13 000 Pa (0,01 à 100 mm de Hg), et préférentiellement allant de 1,3 Pa à 1300 Pa (0,01 à 10 mm de Hg).

En outre, l'huile volatile a généralement un point d'ébullition, mesuré à pression atmosphérique, allant de 150 °C à 260 °C, et de préférence allant de 170 °C à 250 °C.

Avantageusement, la phase grasse comprend de 5 % à 40 % en poids, de préférence allant de 10 % à 35 % en poids, et préférentiellement allant de 15 % à 30 % en poids d'huile(s) volatile(s) par rapport au poids total de la composition.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor ; elle peut contenir des groupes ester, éther, amine, amide.

Par huile siliconée, on entend une huile contenant au moins un atome de silicium, et notamment contenant des groupes Si-O.

Par huile fluorée, on entend une huile contenant au moins un atome de fluor.

### Huiles volatiles additionnelles

L'huile volatile hydrocarbonée utilisable dans l'invention peut être choisie parmi les huiles hydrocarbonées ayant un point éclair allant de 40 °C à 102 °C, de préférence allant de 40 °C à 55 °C, et préférentiellement allant de 40 °C à 50 °C. Comme huiles volatiles hydrocarbonées ayant de 7 à 16 atomes de carbone on peut citer notamment les alcanes linéaires volatils en C7-C14, les alcanes ramifiés en $C_8$-C14 comme les iso-alcanes (appelées aussi isoparaffines) en $C_8$-C14, l'isododécane, l'isodécane, l'isohexadécane et par exemple les huiles vendues sous les noms commerciaux d'Isopars ou de Permetyls, les esters ramifiés en $C_8$-C14 comme le néopentanoate d'iso-hexyle, et leurs mélanges. De préférence, l'huile volatile hydrocarbonée est choisie parmi les huiles volatiles hydrocarbonées ayant de 8 à 16 atomes de carbone et leurs mélanges, en particulier parmi l'isododécane, l'isodécane, l'isohexadécane, et est notamment l'isododécane.

L'huile volatile siliconée utilisable dans l'invention peut être choisie parmi les huiles siliconées ayant un point éclair allant de 40 °C à 102 °C, de préférence ayant un point éclair supérieur à 55 °C et inférieur ou égal à 95 °C, et préférentiellement allant de 65 °C à 95 °C.

Comme huile volatile siliconée, on peut citer les huiles de silicones linéaires ou cycliques ayant de 2 à 7 atomes de silicium, ces silicones comportant éventuellement des groupes alkyle ou alkoxy ayant de 1 à 10 atomes de carbone. Comme exemple d'huile volatile siliconée, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

Comme huile volatile fluorée, on peut citer le nonafluoroéthoxybutane, le nonafluorométhoxybutane, le décafluoropentane, le tétradécafluorohexane, le dodécafluoropentane, et leurs mélanges.

L'huile volatile additionnelle choisie parmi les huiles volatiles hydrocarbonées additionnelles, les huiles volatiles siliconées, les huiles volatiles fluorées, et leurs mélanges peut être présente en une teneur allant de 0 % à 25 % en poids, par rapport au poids total des huiles, en particulier de 0 à 15% en poids, et préférentiellement allant de 0 % à 10 % en poids par rapport au poids total de la composition. Selon un mode particulier, la composition ne comprend pas d'huile volatile additionnelle.

La phase grasse de l'émulsion selon l'invention peut comprendre en outre au moins une huile non volatile.

Huiles non volatiles

Cette huile non volatile ou un de ses mélanges peut être présente en une teneur allant de 0 % à 25 % en poids, par rapport au poids total de l'émulsion, et de préférence allant de 0 à 10 % en poids.

L'huile non volatile peut être choisie parmi les huiles d'origine minérale, animale, végétale ou synthétique, carbonées, hydrocarbonées et/ou siliconées, et leurs mélanges, dans la mesure où elles sont compatibles avec l'utilisation envisagée.

On peut citer les huiles hydrocarbonées non volatiles telles que l'huile de paraffine ou de vaseline, l'isoeicosane, de soja, le perhydrosqualène, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras, tels que le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol.

Comme huile non volatile siliconée, on peut citer les polydiméthylsiloxane (PDMS), éventuellement phénylées telles que les phényltriméthicones, ou éventuellement substitués par des groupements aliphatiques et/ou aromatiques, ou par des groupements fonctionnels tels que des groupements hydroxyle, thiol et/ou amine ; les polysiloxanes modifiés par des acides gras, des alcools gras ou des polyoxyalkylènes, et leurs mélanges.

Selon un mode particulier, la composition pourra comprendre une huile polydiméthylsiloxane (PDMS), étant entendu que le rapport pondéral alcanes linéaires volatils/ PDMS sera supérieur ou égal à 1.

De préférence, ces huiles additionnelles, volatiles ou non volatiles représentent moins de 50% en poids, notamment moins de 40% en poids, voire moins de 30% en poids, moins de 20% en poids, ou encore moins de 10% en poids, ou encore moins de 1% en poids par rapport au poids total des huiles dans la composition.

Ainsi, le rapport pondéral entre les alcanes linéaires volatils en C7-C16 et les huiles additionnelles sera de préférence supérieur ou égal à 1.

Une composition selon l'invention comprendra généralement au moins un agent tensioactif.

## ***AGENTS TENSIOACTIFS***

Les agents tensioactifs utilisables selon l'invention , adaptés aux émulsions E/H, sont notamment choisi(s) parmi les agents tensioactifs amphotères, anioniques, cationiques ou non ioniques, utilisés seuls ou en mélange. Selon un mode préféré, on utilisera des tensioactifs non ioniques.

Les agents tensioactifs sont généralement présents dans la composition, en une teneur pouvant aller de 0,5 à 15 % en poids, en particulier de 1,5 à 5% en poids, par rapport au poids total de la composition.

A titre d'exemple, le ou les tensioactif(s) siliconé(s) peut/peuvent être présent(s) en une teneur allant de 0,5 à 10 % en poids, notamment de 1 à 5 % en poids, par rapport au poids total de la composition.

Selon un autre mode de réalisation particulier, le ou les tensioactif(s) non siliconé(s) peut/peuvent être présent (s) en une teneur allant de 1 à 10 % en poids, notamment de 2 à 8 % en poids, par rapport au poids total de la composition.

Pour les émulsions E/H, on peut utiliser notamment des agents tensioactifs hydrocarbonés ou siliconés.

On peut citer par exemple comme agents tensioactifs hydrocarbonés, les polyesters de polyols comme le PEG-30 dipolyhydroxystearate vendu sous la référence ARLACEL P 135 par la société Uniqema, le polyglyceryl-2 dipolyhydroxystearate vendu sous la référence DEHYMULS PGPH par la société Cognis.

On peut citer par exemple comme agents tensioactifs siliconés les alkyl-diméthicone copolyols tels que le Laurylmethicone copolyol vendu sous la dénomination "Dow Corning 5200 Formulation Aid" par la société Dow Corning et le Cetyl dimethicone copolyol vendu sous la dénomination ABIL EM 90 par la société Goldschmidt, ou le mélange polyglycéryl-4 isostéarate/cétyl diméthicone copolyol/hexyllaurate vendu sous la dénomination ABIL WE 09 par la société Goldschmidt.

On peut y ajouter aussi un ou plusieurs co-émulsionnants. De manière avantageuse, le co-émulsionnant peut être choisi dans le groupe comprenant les esters alkylés de polyol. Comme esters alkylés de polyol, on peut citer notamment les esters de glycérol et/ou de sorbitan et par exemple le polyglyceryl-3 diisostéarate commercialisé sous la dénomination de LAMEFORM TGI par la société Cognis, l'isostéarate de polyglycérol-4, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Selon un mode particulier, la composition de l'invention comprend au moins un tensioactif siliconé choisi parmi les diméthicone copolyols, en particulier le mélange polyglycéryl-4 isostéarate/cétyl diméthicone copolyol/hexyllaurate vendu sous la dénomination ABIL WE 09 par la société Goldschmidt.

La composition selon l'invention pourra également comprendre au moins un agent gélifiant ou structurant de la phase grasse, destiné à augmenter la viscosité de ladite composition.

Selon un mode particulier de l'invention, la composition comprend au moins un gélifiant lipophile.

### *AGENTS GELIFIANTS LIPOPHILES*

Un agent gélifiant convenant à l'invention est avantageusement lipophile. Un gélifiant lipophile peut être minéral ou organique (notamment polymérique).

Le ou les gélifiants lipophiles pourront être présents dans la composition en une teneur allant de 0,1 à 20% en poids, en particulier de 0,2 à 15% en poids, et mieux de 0,25 à 10% en poids par rapport au poids total de ladite composition.

#### *Gélifiants minéraux*

Comme gélifiants lipophiles, on peut citer par exemple les argiles modifiées hydrophobes telles que le silicate de magnésium modifié (Bentone gel VS38 de RHEOX), l'hectorite modifiée par le chlorure de distéaryl diméthyl ammonium (nom CTFA : Disteardimonium hectorite) commercialisé sous la dénomination « Bentone 38 CE » par la société RHEOX.

Comme gélifiant lipophile minéral, on peut citer les argiles éventuellement modifiées comme les hectorites modifiées par un chlorure d'ammonium d'acide gras en $C_{10}$ à $C_{22}$, comme l'hectorite modifiée par du chlorure de distéaryl di-méthyl ammonium telle que, par exemple, celle commercialisée sous la dénomination de Bentone 38V® par la société ELEMENTIS.

#### *Gélifiants organiques polymériques*

Les gélifiants lipophiles organiques polymériques sont par exemple les organopolysiloxanes élastomériques partiellement ou totalement réticulés, de structure tridimensionnelle, comme ceux commercialisés sous les dénominations de KSG6®, KSG16® et de KSG18® par la société SHIN-ETSU, de Trefil E-505C® et Trefil E-506C® par la société DOW-CORNING, de Gransil SR-CYC®, SR DMF10®, SR-DC556®, SR 5CYC gel®, SR DMF 10 gel® et de SR DC 556 gel® par la société GRANT INDUSTRIES, de SF 1204® et de JK 113® par la société GENERAL ELECTRIC ; l'éthylcellulose comme celle vendue sous la dénomination Ethocel® par la société DOW CHEMICAL ; les polycondensats de type polyamide résultant de la condensation entre un acide dicarboxylique comprenant au moins 32 atomes de carbone et un alkylène diamine et en particulier l'éthylène diamine, dans lequel le polymère comprend au moins un groupe acide carboxylique terminal estérifié ou amidifié avec au moins un mono alcool ou une mono amine comprenant de 12 à 30 atomes de carbone linéaires et saturés, et en particulier, les copolymères d'éthylène diamine/dilinoléate de stéaryle tel que celui commercialisé sous la dénomination Uniclear 100 VG® par la société ARIZONA CHEMICAL ; les galactommananes comportant de un à six, et en particulier de deux à quatre, groupes hydroxyle par ose, substitués par une chaîne alkyle saturée ou non, comme la gomme de guar alkylée par des chaînes alkyle en $C_1$ à $C_6$, et en particulier en $C_1$ à $C_3$ et leurs mélanges.

Comme agents gélifiants lipophiles convenant à l'invention peuvent également être cités les copolymères du type polystyrène/polyalkylène, et plus particulièrement les copolymères séquencés de type « dibloc », « tribloc » ou « radial » du type polystyrène/polyisoprène, polystyrène/polybutadiène tels que ceux commercialisés sous la dénomination Luvitol HSB® par la société BASF, du type polystyrène/copoly(éthylène-propylène) tels que ceux commercialisés sous la dénomination de Kraton® par la société KRATON POLYMERS ou encore du type polystyrène/copoly(éthylène-butylène), les mélanges de copolymères tribloc et radial (en étoile) dans l'isododécane tels que ceux commercialisé par la société PENRECO sous la dénomination Versagel® comme par exemple le mélange de copolymère tribloc butylène/éthylène/styrène et de copolymère étoile éthylène/propylène/styrène dans l'isododécane (Versagel M 5960).

Parmi les gélifiants lipophiles pouvant être utilisés dans une composition cosmétique de l'invention, on peut encore citer les esters de dextrine et d'acide gras, tels que les palmitates de dextrine, notamment tels que ceux commercialisés sous les dénominations Rheopearl TL® ou Rheopearl KL® par la société CHIBA FLOUR, les huiles végétales hydrogénées, telles que l'huile de ricin hydrogénée, les alcools gras, en particulier de $C_8$ à $C_{26}$, et plus particulièrement de $C_{12}$ à $C_{22}$, comme par exemple, l'alcool mysritylique, l'alcool cétylique, l'alcool stéarylique ou l'alcool béhénylique.

Selon un mode de réalisation particulièrement préféré, une composition selon l'invention peut comprendre au moins un agent gélifiant lipophile minéral choisi parmi les argiles modifiées hydrophobes.

### *AGENTS STRUCTURANTS LIPOPHILES*

La phase grasse peut également comprendre au moins un agent strucurant lipophile. Le ou les agents structurants lipophiles pourront être présents dans la composition en une teneur allant de 0,05 à 10% en poids, en particulier de 0,1 à 8% en poids, et mieux de 0,2 à 5% en poids par rapport au poids total de ladite composition.

En particulier, ledit agent structurant lipophile peut être choisi parmi au moins une cire, au moins une gomme et/ou au moins un corps gras pâteux, d'origine végétale, animale, minérale ou de synthèse, voire siliconé, et leurs mélanges.

Cires

Parmi les cires solides à température ambiante, susceptibles d'être présentes dans la composition selon l'invention, on peut citer les cires hydrocarbonées telles que la cire d'abeilles, la cire de Carnauba, de Candellila, d'Ouricoury, du Japon, les cires de fibres de liège ou de canne à sucre, les cires de paraffine, de lignite, les cires microcristallines, la cire de lanoline, la cire de Montan, les ozokérites, les cires de polyéthylène, les cires obtenues par synthèse de Fischer-Tropsch, les huiles hydrogénées, les esters gras et les glycérides concrets à 25 °C. On peut également utiliser des cires de silicone, parmi lesquelles on peut citer les alkyl, alcoxy et/ou esters de polyméthylsiloxane. Les cires peuvent se présenter sous forme de dispersions stables de particules colloïdales de cire telles qu'elles peuvent être préparées selon des méthodes connues, telles que celles de "Microemulsions Theory and Practice", L. M. Prince Ed., Academic Press (1977), pages 21-32. Comme cire liquide à température ambiante, on peut citer l'huile de Jojoba.

On peut également mentionner les huiles végétales hydrogénées, telles que l'huile de ricin hydrogénée.

A titre de structurant lipophile convenant également à l'invention, on peut mentionner les alcools gras, en particulier de $C_8$ à $C_{26}$, et plus particulièrement de $C_{12}$ à $C_{22}$.

Selon un mode de réalisation, un alcool gras convenant à l'invention peut être choisi parmi l'alcool myristylique, l'alcool cétylique, l'alcool stéarylique, l'alcool béhénylique.

A titre de structurant lipophile convenant également à l'invention, on peut mentionner les esters d'acide gras et de glycérols, tels que le tristéarate de glycéryle.

Les cires peuvent être présentes à raison de 0,1 % à 10 %, en poids, par rapport au poids total de l'émulsion, et de préférence de 0,1 % à 5 % en poids.

Composés gras pâteux

On peut définir les composés gras pâteux à l'aide d'au moins une des propriétés physico-chimiques suivantes :

- une viscosité de 0,1 à 40 Pa.s (1 à 400 poises), de préférence 0,5 à 25 Pa.s, mesurée à 40 °C avec un viscosimètre rotatif CONTRAVES TV équipé d'un mobile MS-r3 ou MS-r4 à la fréquence de 60 Hz,
- un point de fusion de 25-70 °C, de préférence 25-55 °C.

Les compositions de l'invention peuvent également comprendre au moins une alkyl-, alkoxy- ou phényl-diméthicone telle que, par exemple le produit vendu sous la dénomination de "Abil wax 2440" par la Société GOLDSCHMIDT.
La composition de l'invention peut également comprendre au moins une charge, de nature organique ou minérale.

### *CHARGES*

Par « charge », il faut comprendre les particules incolores ou blanches, solides de toutes formes, qui se présentent sous une forme insoluble et dispersée dans le milieu de la composition. De nature minérale ou organique, elles permettent de conférer à la composition de la douceur, de la matité et de l'uniformité au maquillage.
Les charges peuvent être présentes dans l'émulsion en une teneur allant de 0,1 % à 10 % en poids, par rapport au poids total de l'émulsion, de préférence 0,5 % à 7 %.
Les charges utilisées dans les compositions selon la présente invention peuvent être de formes lamellaires, globulaires, sphériques, de fibres ou de toute autre forme intermédiaire entre ces formes définies.
Les charges selon l'invention peuvent être ou non enrobées superficiellement, et, en particulier, elles peuvent être traitées en surface par des silicones, des acides aminés, des dérivés fluorés ou toute autre substance favorisant la dispersion et la compatibilité de la charge dans la composition.
Parmi les charges minérales utilisables dans les compositions selon l'invention, on peut citer le talc, le mica, la silice, le siloxysilicate de triméthyle, le kaolin, la bentone, le carbonate de calcium et l'hydrogéno-carbonate de magnésium, l'hydroxyapatite, le nitrure de bore, les microsphères de silice creuses (Silice Beads de Maprecos), les microcapsules de verre ou de céramique, les charges à base de silice comme l'Aerosil 200, l'Aerosil 300 ; la Sunsphere H-33, la Sunsphere H-51 commercialisés par Asahi Glass ; le Chemicelen commercialisé par Asahi Chemical ; les composites de silice et de dioxyde de titane, comme la série TSG commercialisée par Nippon Sheet Glass, et leurs mélanges.
Parmi les charges organiques utilisables dans les compositions selon l'invention on peut citer les poudres de polyamide (Nylon® Orgasol de chez Atochem), de poly-b-alanine et polyéthylène, les poudres de polytétrafluoroéthylène (Téflon®), la lauroyl-lysine, l'amidon, les poudres de polymères de tétrafluoroéthylène, les microsphères creuses de polymères, telles l'EXPANCEL (NOBEL INDUSTRIE), les savons métalliques dérivés d'acides organiques carboxyliques ayant de 8 à 22 atomes de carbone, de préférence, de 12 à 18 atomes de carbone, par exemple, le stéarate de zinc, de magnésium ou de lithium, le laurate de zinc, le myristate de magnésium, le Polypore® L 200 (Chemdal Corporation), les microbilles de résine de silicone (Tospearl® de Toshiba, par exemple), les poudres de polyuréthane, en particulier, les poudres de polyuréthane réticulé comprenant un copolymère, ledit copolymère comprenant du triméthylol hexyllactone, comme le polymère d'hexaméthylène di-isocyanate/triméthylol hexyllactone, commercialisé sous la dénomination de PLASTIC POWDER D-400® ou PLASTIC POWDER D-800® par la société TOSHIKI, les microcires de Carnauba, telles que celle commercialisée sous la dénomination de MicroCare 350® par la société MICRO POWDERS, les microcires de cire synthétique, telles que celle commercialisée sous la dénomination de MicroEase 114S® par la société MICRO POWDERS, les microcires constituées d'un mélange de cire de Carnauba et de cire de polyéthylène, telles que celles commercialisées sous les dénominations de Micro Care 300® et 310® par la société MICRO POWDERS, les microcires constituées d'un mélange de cire de Carnauba et de cire synthétique, telles que celle commercialisée sous la dénomination Micro Care 325® par la société MICRO POWDERS, les microcires de polyéthylène, telles que celles commercialisées sous les dénominations de Micropoly 200®, 220®, 220L® et 250S® par la société MICRO POWDERS ; les fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser. les fibres ont une longueur allant de 1 μm à 10 mm, de préférence de 0,1 mm à 5 mm et mieux de 0,3 mm à 3 mm. Leur section peut être comprise dans un cercle de diamètre allant de 2 nm à 500 μm, de préférence allant de 100 nm à 100 μm et mieux de 1 μm à 50 μm. A titre de fibres utilisables dans les compositions selon l'invention, on peut citer les fibres non rigides telles que les fibres de polyamide (Nylon®) ou les fibres rigides telles que les fibres de polyimide-amide comme celles vendues sous les dénominations KERMEL®, KERMEL TECH® par la société RHODIA ou de poly-(p-phénylène-téréphtalamide) (ou d'aramide) notamment vendues sous la dénomination Kevlar ® par la société DUPONT DE NEMOURS,
et leurs mélanges.

### Adjuvants cosmétiques

Les compositions de l'invention peuvent contenir en outre un ou plusieurs des adjuvants habituels dans les domaines cosmétique et dermatologique, tels que des agents hydratants ; des émollients ; des actifs hydrophiles ou lipophiles ; des agents anti-radicaux libres ; des séquestrants ; des antioxydants ; des conservateurs ; des agents alcanisants ou acidifiants ; des parfums ; des gélifiants hydrophiles ; des agents filmogènes, en particulier des polymères filmogènes (pour des compositions dans un axe tenue) ; des filtres solaires organiques ou physiques, des colorants hydrosolubles ou liposolubles; et leurs mélanges. Les quantités de ces différents adjuvants sont celles classiquement utilisées dans les fonds de teint.

Bien entendu, l'homme de l'art veillera à choisir le ou les éventuels adjuvants ajoutés à la composition selon l'invention de manière telle que les propriétés avantageuses attachées intrinsèquement à la composition conforme à l'invention ne soient pas, ou substantiellement pas, altérées par l'addition envisagée.

Ces adjuvants sont généralement présents dans la composition en une teneur allant de 0,01 à 20 % en poids, de préférence de 0,1 à 10 % en poids par rapport au poids total de ladite composition.

L'invention est illustrée dans les exemples présentés ci-après à titre illustratif et non limitatif du domaine de l'invention :

Sauf indication contraire, les valeurs dans les exemples ci-dessous sont exprimées en % en poids par rapport au poids total de la composition.

## EXEMPLES

### Exemple 1 : Propriétés cosmétiques des compositions selon l'invention

**a) Composition et préparation des formules testées**

| Ingrédients | Formule A (comparatif) | Formule B (invention) | Formule C (invention) |
|---|---|---|---|
| Sulfate de magnésium | 1,50 | 1,50 | 1,50 |
| Hectorite modifiée distearyl dimethyl ammonium (BENTONE 38 VCG de Elementis) | 1 | 1 | 1 |
| MICROSPHERES (COPOLYMERE CHLORURE DE VINYLIDENE / ACRYLONITRILE / METHACRYLATE DE METHYLE) EXPANSEES PAR ISOBUTANE (Expancel 551 DE 40 D42 de Expancel) | 0,60 | 0,60 | 0,60 |
| Dioxyde de titane | 21,20 | 21,20 | 21,20 |
| Oxyde de fer rouge | 0,89 | 0,89 | 0,89 |
| Oxyde de fer noir | 0,33 | 0,33 | 0,33 |
| Oxyde de fer jaune | 2,58 | 2,58 | 2,58 |
| Tristearin acetylated glycol stearate (Unitwix de United Guardian) | 0,30 | 0,30 | 0,30 |
| Carboxymethylcellulose de sodium | 0,50 | 0,50 | 0,50 |
| Cyclopentasiloxane | **15,30** | | |
| Polyglyceryl-4 isostearate cetyl PEG/PPG-10/1 hexyl laurate (ABIL WE 09 de Evonik Goldschmidt) | 9 | 9 | 9 |
| POLY DIMETHYLSILOXANE (VISCOSITE: 5 CST) (XIAMETER PMX-200 SILICONE FLUID 5CS de Dow Corning) | **6,60** | **6,60** | |

(suite)

| Ingrédients | Formule A (comparatif) | Formule B (invention) | Formule C (invention) |
|---|---|---|---|
| Glycérine | 5 | 5 | 5 |
| Eau de La Roche-Posay | 31,70 | 31,70 | 31,70 |
| Ethylhexyl glycérine | 0,50 | 0,50 | 0,50 |
| Pentylene glycol | 3 | 3 | 3 |
| Mélange majoritaire de nundécane : n-tridécane dans lequel le n-undécane est majoritaire dans le mélange* | | **15,30** | **21,90** |
| * tel que préparé selon la demande WO2008/155059 | | | |

Les compositions sont préparées comme suit :

Faire chauffer le mélange de tensio-actifs sur un bain-marie, dans un bécher à 75°C. Une fois le mélange fondu (vers 65°C), le placer sur plaque chauffante (consigne 100°C) et
sous agitation à la turbine.

Empâter les pigments avec une partie de la phase grasse, passer 3 fois à la broyeuse tricylindre. Utiliser le reste de la phase grasse pour empâter les charges.

A 65°C, ajouter la bentone dans le mélange de tensio-actifs chauffé et turbiner ; puis additionner le mélange pigmentaire et la charge empâtée à 40°C et laisser refroidir sous Moritz.

Laisser refroidir à 25°C.

En parallèle, mélanger tous les constituants de la phase aqueuse, tout peser et ajouter l'eau, puis passer sur plaque chauffante pour monter à 70°C et sous Rayneri. Y saupoudrer la cellulose et agiter jusqu'à solubilisation totale. Emulsionner 10 min à température ambiante sous turbine en ajoutant la phase aqueuse dans la phase grasse préalablement préparée.

La même émulsion a été réalisée avec 30% de pigments (oxydes de fer + dioxyde de titane) au lieu de 25%, respectivement avec une huile siliconée volatile (formule D) et avec au moins un alcane linéaire volatile selon l'invention (formule E).

| Ingrédients | Formule D (comparatif) | Formule E (invention) |
|---|---|---|
| Sulfate de magnésium | 1,50 | 1,50 |
| Hectorite modifiée distearyl dimethyl ammonium (BENTONE 38 VCG de Elementis) | 1 | 1 |
| MICROSPHERES (COPOLYMERE CHLORURE DE VINYLIDENE / ACRYLONITRILE / METHACRYLATE DE METHYLE) EXPANSEES PAR ISOBUTANE (Expancel 551 DE 40 D42 de Expancel) | 0,60 | 0,60 |
| Dioxyde de titane | 25,06 | 25,06 |
| Oxyde de fer rouge | 1,16 | 1,16 |
| Oxyde de fer noir | 0,43 | 0,43 |
| Oxyde de fer jaune | 3,35 | 3,35 |
| Tristearin acetylated glycol stearate (Unitwix de United Guardian) | 0,30 | 0,30 |
| Carboxymethylcellulose de sodium | 0,50 | 0,50 |
| Cyclopentasiloxane | **16,9** | |

(suite)

| Ingrédients | Formule D (comparatif) | Formule E (invention) |
|---|---|---|
| Polyglyceryl-4 isostearate cetyl PEG/PPG-10/1 hexyl laurate (ABIL WE 09 de Evonik Goldschmidt) | 9 | 9 |
| POLY DIMETHYLSILOXANE (VISCOSITE: 5 CST) (XIAMETER PMX-200 SILICONE FLUID 5CS de Dow Corning) | **0** | **0** |
| Glycérine | 5 | 5 |
| Eau de La Roche-Posay | 30,70 | 30,70 |
| Ethylhexyl glycérine | 0,50 | 0,50 |
| Pentylene glycol | 3 | 3 |
| Mélange majoritaire de n-undécane : n-tridécane dans lequel le n-undécane est majoritaire dans le mélange* | | **16,9** |
| * tel que préparé selon la demande WO2008/155059 | | |

**b) Stabilité**

On évalue la stabilité des compositions A à E par observation macroscopique et microscopique, après 24 heures, 1 mois et 2 mois à température ambiante, 45°C et après 3 cycles de température de -20 °C à +20 °C dans une étuve .(ex : Vötch VT4004).
Chacun des cycles dure 24 heures et comprend les étapes suivantes 6 heures à 20 °C, puis 6 heures de descente en température jusqu'à -20 °C, puis 6 heures à une température de - 20 °C, et enfin 6 heures de remontée en température jusqu'à 20 °C. Après chaque cycle, les aspects macroscopiques et microscopiques de la composition sont évalués.
Au bout de 3 cycles, la composition ne doit pas présenter de modifications d'aspect macroscopique : elle doit rester lisse et homogène, sans relargage, sans séparation de phase et sans changement de couleur.
La composition est observée au microscope entre lame et lamelle, à un grossissement X10. Son aspect microscopique doit rester proche de l'aspect initial : on ne doit pas observer, en particulier, de dégradation de l'émulsion (fond d'émulsion plus grossier, coalescence traduite par la présence de nombreuses grosses gouttes, modification des bords de préparation, présence de cristaux).
L'essai est considéré comme stable si l'émulsion reste fine, sans lâchage sur les bords, sans coalescence.
La composition A (comparatif) est instable après 3 cycles de température.
Les compositions B et C (invention) sont stables après 24h, 1 mois et 2 mois.
La composition D (comparatif) résulte en un mélange inhomogène de pigments, de phase aqueuse et de phase huileuse, tandis que la composition E (invention) reste stable après 24H, 1 mois et 2 mois.

**c) Evaluation sensorielle**

Protocole

Par ailleurs, une évaluation sensorielle desdites compositions A à C a été réalisée sur un panel de 15 personnes expertes utilisatrices de fond de teint. Après application standardisée, chaque personne évalue la perception de ladite composition au moment de l'application et en termes de résultat maquillage suivant certains critères prédéfinis sur une échelle normée.
A l'application, chaque modèle évalue notamment les paramètres suivants : fluidité, glissant, toucher poudreux, facilité d'application, toucher gras.
Après application, chaque modèle évalue notamment le résultat maquillage : fini poudreux, couvrance, uniformité.

Résultats

Comparées à la formule A (comparative), les émulsions B et C obtenues sont crémeuses, faciles d'application, non grasses, permettent de couvrir les imperfections cutanées d'intensité prononcée et laissent un film léger, doux et non collant après application.

**Exemple 2 : Propriétés de couvrance des compositions selon l'inventioon**

Les supports utilisés pour la mesure de couvrance des formules A à C décrites ci-dessus sont des plaques de Bioskin de couleur noire et blanche (fournisseur Maprecos). Les compositions A à C préparées à l'exemple 1 sont appliquées au doigtier à raison de 32mg sur une surface de 5cm de diamètre, puis séchés pendant 90 minutes. En particulier, l'épaisseur appliquée est de 15µm.

Les spectres de réflectance sont acquis à l'aide d'un spectrocolorimètre MINOLTA 3700-d (géométrie de mesure diffuse et observation D65/10°, mode composante spéculaire exclue) sur les fonds noir et blanc (3 mesures sur chaque application).

Les spectres sont exprimés en coordonnées colorimétriques dans l'espace CIEYxy1931 au sens de la Commission Internationale de l'Eclairage.

Le contraste ratio CR, ou couvrance, est calculé en faisant la moyenne arithmétique de Y sur fond noir, divisée par la valeur moyenne de Y sur fond blanc, multiplié par 100.

$$CR = [Y(fond\ noir) / Y\ (fond\ blanc)]x100$$

Formule A (comparatif) : CR = 86

Formule B (contenant 15,3% d'alcane linéaire volatil) : CR = 81

Formule C (contenant 21,9% d'alcane linéaire volatil) : CR = 76

Les 3 formules sont dans le domaine de la très haute couvrance car proches de 100.

De façon avantageuse, les compositions B et C selon l'invention donnent un résultat maquillage plus naturel que la composition A (comparative) : les traits sont moins marqués et les imperfections de relief (type squames, pores dilatés ou zones sèches) sont moins visibles.

Ces résultats montrent que l'utilisation d'alcanes linéaires volatiles en C7-C14 selon l'invention, dans une architecture E/H, permettent d'intégrer une forte teneur en pigments (25 et 30% testés), avec des propriétés de très haute couvrance, tout conférant au produit des propriétés cosmétiques à l'application améliorées, comparativement à la même architecture mais avec des huiles volatiles siliconées.

**Exemple 3 : Correcteur de teint**

| Ingrédients | Formule F (invention) |
|---|---|
| Sulfate de magnésium | 1,50 |
| Hectorite modifiée distearyl dimethyl ammonium (BENTONE 38 VCG de Elementis) | 1 |
| MICROSPHERES (COPOLYMERE CHLORURE DE VINYLIDENE / ACRYLONITRILE / METHACRYLATE DE METHYLE) EXPANSEES PAR ISOBUTANE (Expancel 551 DE 40 D42 de Expancel) | 0,60 |
| Dioxyde de titane | 21,20 |
| Oxyde de fer rouge | 0,89 |
| Oxyde de fer noir | 0,33 |
| Oxyde de fer jaune | 2,58 |
| Tristearin acetylated glycol stearate (Unitwix de United Guardian) | 0,30 |
| Carboxymethylcellulose de sodium | 0,50 |
| Polyglyceryl-4 isostearate cetyl PEG/PPG-10/1 hexyl laurate (ABIL WE 09 de Evonik Goldschmidt) | 9 |
| Glycérine | 5 |
| Eau de La Roche-Posay | 31,70 |
| Ethylhexyl glycérine | 0,50 |

(suite)

| Ingrédients | Formule F (invention) |
|---|---|
| Pentylene glycol | 3 |
| Dodécane (Parafol C12-97 de Sasol) | 21,9 |

La composition F dérive de la composition C selon l'invention dans laquelle le mélange majjoritaire de undécane : tridécane a été remplacé par un autre alcane linéaire volatil selon l'invention, le dodécane.

Les compositions selon l'invention sont appliquées de façon uniforme sur la peau du visage, ou par petites touches pour camoufler des imperfections cutanées, en particulier au niveau des défauts de couleur de la peau.

**Exemple 4 : Effet des alcanes linéaires volatils sur les propriétés cosmétiques des compositions selon l'invention comparativement à d'autres huiles hydrocarbonées volatiles**

Les 3 compositions suivantes sont préparées comme décrit à l'exemple 1. La composition C (invention) est décrite à l'exemple 1 et les compositions G et H (comparatifs) comprennent des huiles hydrocarbonées volatiles distinctes des alcanes linéaires volatils selon l'invention.

| Ingrédients | Formule C (invention) | Formule G (comparatif) | Formule H (comparatif) |
|---|---|---|---|
| Sulfate de magnésium | 1,50 | 1,50 | 1,50 |
| Hectorite modifiée distearyl dimethyl ammonium (BENTONE 38 VCG de Elementis) | 1 | 1 | 1 |
| MICROSPHERES (COPOLYMERE CHLORURE DE VINYLIDENE / ACRYLONITRILE / METHACRYLATE DE METHYLE) EXPANSEES PAR ISOBUTANE (Expancel 551 DE 40 D42 de Expancel) | 0,60 | 0,60 | 0,60 |
| Dioxyde de titane | 21,20 | 21,20 | 21,20 |
| Oxyde de fer rouge | 0,89 | 0,89 | 0,89 |
| Oxyde de fer noir | 0,33 | 0,33 | 0,33 |
| Oxyde de fer jaune | 2,58 | 2,58 | 2,58 |
| Tristearin acetylated glycol stearate (Unitwix de United Guardian) | 0,30 | 0,30 | 0,30 |
| Carboxymethylcellulose de sodium | 0,50 | 0,50 | 0,50 |
| Isohexadécane | | **21,90** | |
| Isododécane | | | **21,90** |
| Polyglyceryl-4 isostearate cetyl PEG/PPG-10/1 hexyl laurate (ABIL WE 09 de Evonik Goldschmidt) | 9 | 9 | 9 |
| Glycérine | 5 | 5 | 5 |
| Eau de La Roche-Posay | 31,70 | 31,70 | 31,70 |
| Ethylhexyl glycérine | 0,50 | 0,50 | 0,50 |

(suite)

| Ingrédients | Formule C (invention) | Formule G (comparatif) | Formule H (comparatif) |
|---|---|---|---|
| Pentylene glycol | 3 | 3 | 3 |
| Mélange majoritaire de n-undécane : n-tridécane dans lequel le n-undécane est majoritaire dans le mélange* | 21,90 | | |
| * tel que préparé selon la demande WO2008/155059 | | | |

On a mesuré la viscosité des compositions ainsi préparées et évalué la stabilité et l'aspect sensoriel de chacune selon les protocoles respectifs décrits à l'exemple 1 ci-dessus.

Viscosité

La viscosité des compositions est mesurée après 24h au repos sur un Rhéomat 180 (Société LAMY), au Mobile M3 à 25°C, après 10min de cisaillement à 200trs/min On a obtenu les viscosités suivantes :

| | |
|---|---|
| Composition C (invention) : | 25uD , soit 8.6 poises |
| Composition G (comparatif isohexadécane) : | 51uD, soit 19.5 poises |
| Composition H (comparatif isododécane) : | 31 uD, soit 11.1 poises |

Stabilité

On n'observe pas de différence notable en terme de stabilité entre ces 3 compositions.

Evaluation sensorielle

En revanche, l'évaluation sensorielle sur modèles de ces 3 compositions est très en faveur de la composition C selon l'invention. En effet :

- la composition G (comparatif avec isohexadécane) présente une viscosité deux fois plus importante par rapport à la composition C (selon l'invention), ce qui rend son application moins facile car le produit est plus pâteux et délicat à étaler. Le dépôt sur la peau est par ailleurs collant avec la composition G, contrairement au dépôt avec la composition C selon l'invention.
- la composition H (comparatif avec isododécane) présente une viscosité un peu plus importante que celle de la composition C ; elle s'applique facilement au tout départ mais compte tenu de la grande volatilité de l'isododécane, le 'playtime' du produit (temps dont dispose l'utilisatrice à l'application pour travailler la composition et homogénéiser le rendu maquillage) est très court, ne permettant pas l'obtention d'un dépôt homogène après évaporation de l'isododécane : le dépôt est comme figé. Comme pour la composition G, le dépôt sur la peau est par ailleurs collant, contrairement au dépôt avec la composition C selon l'invention.

Ces résultats confirment que l'utilisation des alcanes linéaires volatiles dans une émulsion eau-dans-huile comprenant un taux important de matières colorantes pulvérulentes, permet d'obtenir une texture crémeuse, facile d'application, permettant de couvrir de façon homogène les imperfections cutanées en laissant un film léger et non collant après application, comparativement aux mêmes architectures de compositions avec d'autres huiles hydrocarbonées volatiles (ex : isododécane, isohexadécane).

**Revendications**

1. Composition cosmétique colorée de maquillage des matières kératiniques, en particulier la peau, sous la forme d'une émulsion eau-dans huile comprenant, dans un milieu physiologiquement acceptable:

(i) au moins 18% en poids de matières colorantes pulvérulentes, par rapport au poids total de la composition,

(ii) au moins un ou plusieurs alcanes linéaires volatils notamment en C7-C14.

**2.** Composition selon la revendication précédente, **caractérisée en ce qu**'elle comprend au moins 20% en poids, de préférence au moins 25% en poids, voire au moins 30% en poids de matières colorantes pulvérulentes, par rapport au poids total de ladite composition.

**3.** Composition selon l'une quelconque des revendications précédentes, **caractérisée en ce qu**'elle comprend au moins 50 % en poids d'alcane(s) linéaire(s) volatil(s) par rapport à la teneur totale en huile(s) volatile(s) de la composition.

**4.** Composition selon l'une quelconque des revendications précédentes, **caractérisée en ce qu**'elle comprend au moins 90%, voire 100 % en poids d'alcane(s) linéaire(s) volatil(s) par rapport à la teneur totale en huile(s) volatile(s).

**5.** Composition selon l'une quelconque des revendications précédentes, **caractérisée en ce que** ledit alcane linéaire volatil comprend de 7 à 14 atomes de carbone, en particulier de 9 à 14 atomes de carbone, et plus particulièrement de 11 à 14 atomes de carbone.

**6.** Composition selon l'une quelconque des revendications précédentes, **caractérisée en ce que** l'alcane linéaire volatil est choisi parmi le n-heptane, le n-octane, le n-nonane, le n-undécane, le n-dodécane, le n-tridécane, le n-tétradécane, et leurs mélanges.

**7.** Composition selon la revendication précédente, **caractérisée en ce qu**'elle comprend au moins du dodécane.

**8.** Composition selon l'une quelconque des revendications précédentes, **caractérisée en ce qu**'elle comprend au moins deux alcanes linéaires volatiles distincts, différant entre eux d'un nombre de carbone n d'au moins 1, en particulier différant entre eux d'un nombre de carbone de 1 ou de 2.

**9.** Composition selon l'une quelconque des revendications précédentes, **caractérisée en ce qu**'elle comprend un mélange d'au moins deux alcanes linéaires volatiles comprenant

- de 50 à 90% en poids, de préférence de 55 à 80% en poids, préférentiellement encore de 60 à 75% en poids d'alcane linéaire volatil en Cn avec n allant de 7 à 14
- de 10 à 50% en poids, de préférence de 20 à 45% en poids, de préférence de 24 à 40% en poids, d'alcane linéaire volatil en Cn+x avec x supérieur ou égal à 1, de préférence x=1 ou x=2, avec n+x compris entre 8 et 14, par rapport au poids total des alcanes dans ledit mélange.

**10.** Composition selon la revendication précédente, **caractérisée en ce qu**'elle comprend un mélange n-undécane : n-tridécane (C11/C13) comprenant

- de 55 à 80% en poids, de préférence de 60 à 75% en poids d'alcane linéaire volatile en C11 (n-undécane)
- de 20 à 45% en poids, de préférence de 24 à 40% en poids d'alcane linéaire volatile en C13 (n-tridécane)
par rapport au poids total des alcanes dans ledit mélange.

**11.** Composition selon l'une quelconque des revendications précédentes, **caractérisée en ce qu**'elle comprend de 5 % à 50 % en poids d'alcane(s) linéaire(s) volatil(s), en particulier de 8 à 40 % en poids d'alcane(s) linéaire(s) volatil (s), et plus particulièrement de 10 à 30 % en poids d'alcane(s) linéaire(s) volatil(s) notamment en C7-C14 par rapport au poids total de la composition.

**12.** Composition selon l'une quelconque des revendications précédentes, **caractérisé en ce qu**'elle comprend en outre au moins un tensioactif siliconé.

**13.** Composition selon l'une quelconque des revendications précédentes, **caractérisée en ce qu**'il s'agit d'un fond de teint ou d'un correcteur de teint.

**14.** Procédé cosmétique de maquillage des matières kératiniques, en particulier la peau, comprenant l'application sur lesdites matières d' une composition telle que définie selon l'une quelconque des revendications 1 à 13.

**15.** Procédé cosmétique selon la revendication précédente, destiné à camoufler les imperfections cutanées de la peau, en particulier les défauts de couleur de la peau.

Europäisches
Patentamt

European
Patent Office

Office européen
des brevets

**RAPPORT DE RECHERCHE EUROPEENNE**

Numéro de la demande

EP 10 19 4890

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | US 5 948 393 A (TOMOMASA SATOSHI [JP] ET AL) 7 septembre 1999 (1999-09-07) * colonne 2, ligne 53 - colonne 3, ligne 7 * * colonne 4, ligne 6 - ligne 15 * * exemples 10,11; tableau 3 * ----- | 1-15 | INV. A61K8/06 A61K8/31 A61Q1/08 |
| Y | DE 200 09 445 U1 (SCHWAN STABILO COSMETICS GMBH [DE]) 17 août 2000 (2000-08-17) * page 4, alinéa 2 - page 8, alinéa 1 * * page 10, alinéa 3 - page 11, alinéa 2; revendications; exemples 25,26 * ----- | 1-15 | |
| Y | WO 2009/082565 A1 (AVON PROD INC [US]; MAITRA PRITHWIRAJ [US]; RANADE RAHUL A [US]; GLYNN) 2 juillet 2009 (2009-07-02) * alinéa [0012] - alinéa [0024]; revendications * * alinéa [0054] - alinéa [0065] * * alinéa [0077] - alinéa [0083]; exemple 3 * ----- | 1-15 | |
| Y | EP 2 111 847 A1 (COGNIS IP MAN GMBH [DE]) 28 octobre 2009 (2009-10-28) * alinéa [0002] - alinéa [0046] * * alinéa [0104] - alinéa [0122] * * alinéa [0151] - alinéa [0157] * * Exemple de préparation 2; alinéa [0180] - alinéa [0185] * * alinéa [0272] - alinéa [0273] * * alinéa [0325] - alinéa [0333] * ----- -/-- | 1-15 | DOMAINES TECHNIQUES RECHERCHES (IPC) A61K A61Q |

Le présent rapport a été établi pour toutes les revendications

| Lieu de la recherche | Date d'achèvement de la recherche | Examinateur |
|---|---|---|
| Munich | 27 juin 2011 | Loloiu, Teodora |

EPO FORM 1503 03.82 (P04C02)

Europäisches
Patentamt

European
Patent Office

Office européen
des brevets

**RAPPORT DE RECHERCHE EUROPEENNE**

Numéro de la demande

EP 10 19 4890

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | DE 10 2008 012457 A1 (COGNIS IP MAN GMBH [DE]) 24 décembre 2008 (2008-12-24) <br> * alinéa [0002] - alinéa [0052] * <br> * alinéa [0080] - alinéa [0100] * <br> * alinéa [0117] - alinéa [0122] * <br> * Exemple de préparation 2; page 52 * <br> * page 61 * <br> ----- | 1-15 | |
| A | WO 01/91704 A1 (PROCTER & GAMBLE [US]; TANIGUCHI TOSHIYA [JP]; TANAKA KOJO [JP]) 6 décembre 2001 (2001-12-06) <br> * page 2, ligne 15 - page 24, ligne 13; revendications; exemples 1-5 * <br> ----- | 1-15 | |

DOMAINES TECHNIQUES
RECHERCHES (IPC)

Le présent rapport a été établi pour toutes les revendications

| Lieu de la recherche | Date d'achèvement de la recherche | Examinateur |
|---|---|---|
| Munich | 27 juin 2011 | Loloiu, Teodora |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**EP 2 351 552 A1**

**ANNEXE AU RAPPORT DE RECHERCHE EUROPEENNE
RELATIF A LA DEMANDE DE BREVET EUROPEEN NO.**

EP 10 19 4890

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

27-06-2011

| Document brevet cité au rapport de recherche | | Date de publication | Membre(s) de la famille de brevet(s) | | Date de publication |
|---|---|---|---|---|---|
| US 5948393 | A | 07-09-1999 | AUCUN | | |
| DE 20009445 | U1 | 17-08-2000 | AUCUN | | |
| WO 2009082565 | A1 | 02-07-2009 | AR | 069794 A1 | 17-02-2010 |
| | | | CA | 2707942 A1 | 02-07-2009 |
| | | | CN | 101903005 A | 01-12-2010 |
| | | | EP | 2229132 A1 | 22-09-2010 |
| | | | JP | 2011507865 A | 10-03-2011 |
| | | | US | 2010266648 A1 | 21-10-2010 |
| EP 2111847 | A1 | 28-10-2009 | DE | 102008017031 A1 | 24-12-2008 |
| DE 102008012457 | A1 | 24-12-2008 | AUCUN | | |
| WO 0191704 | A1 | 06-12-2001 | AU | 5457500 A | 11-12-2001 |
| | | | AU | 2000254575 B2 | 19-08-2004 |
| | | | CA | 2409616 A1 | 06-12-2001 |
| | | | CN | 1454071 A | 05-11-2003 |
| | | | JP | 2003534361 A | 18-11-2003 |
| | | | MX | PA02011924 A | 22-04-2003 |

Pour tout renseignement concernant cette annexe : voir Journal Officiel de l'Office européen des brevets, No.12/82

23

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 542669 A
- EP 787730 A
- EP 787731 A
- WO 9608537 A
- EP 1086683 A

- WO 06013413 A
- WO 2007068371 A
- WO 2008155059 A

**Littérature non-brevet citée dans la description**

- Microemulsions Theory and Practice. Academic Press, 1977, 21-32